(19) **Europäisches Patentamt**

**European Patent Office**

**Office européen des brevets**

(11) **EP 1 013 296 A1**

(12) **EUROPEAN PATENT APPLICATION**

(43) Date of publication:
**28.06.2000   Bulletin 2000/26**

(51) Int Cl.⁷: **A61M 5/19**, A61M 5/315

(21) Application number: **98124364.5**

(22) Date of filing: **22.12.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant: **WINSEL, August, Prof. Dr.
D-65779 Kelkheim (DE)**

(72) Inventors:
• **Winsel, August Dr.
65779 Kelkheim (DE)**

• **Forrester, Ian Terence
Christchurch (NZ)**

(74) Representative: **Specht, Peter, Dipl.-Phys. et al
Loesenbeck, Stracke, Loesenbeck,
Patentanwälte,
Jöllenbecker Strasse 164
33613 Bielefeld (DE)**

(54) **Controlled and regulated conveying of fluid mediums by electrochemical gas generation**

(57)   An apparatus for conveying successively two fluid mediums, especially two drugs, to a human or to an animal client comprising a cylinder 12 with a first piston 9 near to the mouth 1 and a second piston 10 near to the end-plate 13, thus defining three partitions within the cylinder: ⇒ partition one is situated between mouth 1 and piston 9 and contains the first medium, ⇒ partition two is situated between piston 9 and piston 10 and contains the second medium, ⇒ partition three is situated between piston 10 and endplate 13 and takes up the driving gas that is generated by an electrolytic process from gas cells. Piston 9 contains a by-pass 51 and a valve 52 within by-pass 51 for the flow of the second medium through piston 9.

FIG 1

**Description**

SUMMARY OF THE INVENTION

The present invention relates to the conveying of fluids to human or animal clients, and, in particular, to the conveying of fluids using gas evolution cells. It also relates to the conveying of fluids for technical processes. The invention relates chiefly to devices like syringes for the application of fluids to human or animal clients using gas evolution cells.

DESCRIPTION OF THE FORMER ART

German patent DE-PS 2 139 771 and co-pending Canadian patent 961,420 describe an automatic press for lubrication purposes that makes use of the hydrogen evolution of a metallic corrosion element. According to German patent DE-PS 35 32 335.3 and US-PS 5,242,565 it is known in the art to convey technical or industrial fats in ball and roller bearings using fat injectors powered by hydrogen evolution cells. There are also infusors for injection or infusion of medical liquids, e.g. DE-PS 41 21 351 C2, which are driven mechanically by gas evolution cells. In such devices, a constant or regulated electric current in the gas evolution cell generates a gas stream that is proportional to the electric current. The generated gas displaces a piston and the medium to be conveyed within the working space of a cylinder, usually a paste or a liquid. Since the gas evolution is proportional to the cell current, such devices can be regulated and controlled very simply by changing the dosing resistance of the cell.

In the hydrogen evolution cell described in German patent DE-PS 35 32 335.3 and US-PS 5,5242,565, the hydrogen is generated by precipitation on a cathode with a hydrophilic / hydrophobic pore structure. The anode is zinc. The electrolyte is a potassium and / or sodium hydroxide solution. The gas evolution is limited by confining the electrochemical processes to the electrode or can be finished as described by DE-PS 41 21 351 C2 by action of the piston when it reaches it's end-position. The cell current, the highest possible gas generation rate, and the maximal conveying rate of paste or liquid are all determined by this gas evolution. For higher conveying rates, larger gas evolution cells may be constructed and used. Also cells for parallel gas generation can be connected in scries by known methods. The increase and multiplication of the gas generation rate is the task of DE-PS 196 19376 Al.and co-pending US-PS 5,407,555 which both describe the device called gas multiplier. The aspects and advantages of this invention will become apparent from the detailed description of the new method and the new device for the consecutive application of two fluids to a client with an intermediate pause using one single syringe in an uninterrupted process.

DESCRIPTION OF THE INVENTION

The present invention is directed to a process and apparatus for conveying two fluids, especially two drugs, to a client. In certain applications a first drug is applied during several hours of infusion. After a pause a second drug must be applied to the client. Today, this process requires the application of the same procedure twice, which makes it expensive. This invention provides a new gas driven apparatus and a new procedure to fulfil the successive conveying of two different media with an intermediate pause to the client without interrupting the process after it has been started. The gas for the total injection procedure is generated by using one or more gas evolution cells. The first medium is conveyed by action of the generated gas on a combination of two pistons and the second medium between the two pistons. The infusion of the second medium follows after a pause by the same process of gas generation.

DESCRIPTION OF THE DRAWINGS

FIG. 1 shows an preferred embodiment of a syringe according to the invention.
FIG. 2 displays details of the two pistons of the device in FIG. 1.
FIG. 3 shows the mouth region of the syringe with the knife 31.
FIG. 4 displays the infusion process according to the invention.

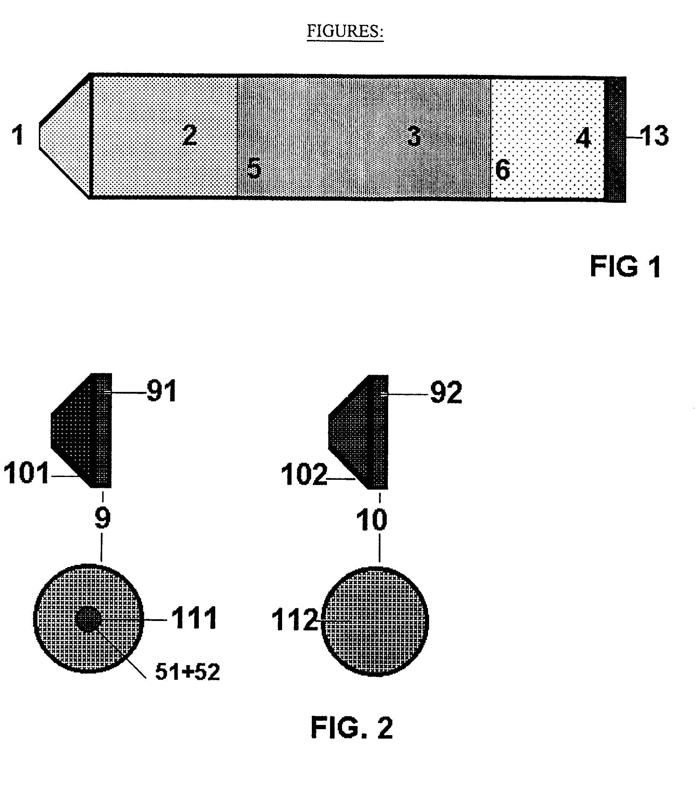

FIG. 1 shows an preferred embodiment of the invention. The apparatus comprises a cylinder 12 with an outlet opening 1 at the top and a bottom part 13 at the end closing the cylinder. By one piston on position 5 and another one on position 6 the cylinder is separated into three partitions: Partition 2 between land 5 contains the first medium to be convey to the client, partition 3 between 5 and 6 contains the second medium, partition 4 between level 6 and bottom 13 contains the pressurised gas that is generated electrochemically in the bottom part 13.

In FIG. 2 the two pistons - 9 in position 5 and 10 in position 6 - are described. As an example, each piston may consist of a skeleton, 91 respective 92, made from a plastic material or from a metal or metal net. This skeleton bears a cloth, 101 respective 102, made from rubber, PVC, PTFE or a similar elastic material to tighten the piston in the cylinder. The piston on position 5 has the cross-section shown as 111. The piston on position 6 with the skeleton 92 and the cloth 102 has the cross-section shown as 112. According to the invention, the piston on position 5 nearest to the opening 1 comprises a by-pass 51 that contains a valve 52. According to the invention, valve 52 is closed as long as compartment 2 still contains fluid amounts of the first medium. Thereafter, valve 52 opens the by-pass for the flow of the second medium in compartment 3 after medium 1 has been conveyed through opening 1 completely and the piston 9 has reached it's end-position.

To guaranty safe operation, endplate 13 may contain a safety-vent that opens if the pressure, $P_4$, of the gas in compartment 4 against the outer atmosphere, $P_o$, exceeds a certain pressure difference $P_4 - P_o >= \Delta\pi$. Valve 52 in by-pass 51 is designed to open, if the pressure difference $P_3 - P_1$ exceeds a given value $\Delta\pi_v$ or if the piston 9 is in it's end position. For the pressure values within the different compartments it follows

$$P_o < P_1 < P_2 < P_3 < P_4 < P_o + \Delta\pi$$

Other aspects, features, and advantages of the present invention will become more fully apparent from the following detailed description, the appended claims, and the accompanying drawings in which FIG. 1 illustrates an apparatus for executing a conveying process according to one embodiment of the present invention.

It is known in the art that hydrogen and oxygen gas can be liberated from water by electrolysis. In DE-PS 35 32 335.3 and US-PS 5,5242,565 electrochemical cells are described that liberate hydrogen or oxygen if a direct current, i, is forced to flow through the cell. The gas rate, that is the amount of gas liberated during one unit of time, t, is constant at constant current. At constant temperature, T=const., the rate of the liberated gas volume, v, is given by this equation

$$dv/dt = (RT/P_4) * i/z/F.$$

In this equation, R= 8,3145 J/mol/K is the gas constant, F=96500 As/mol is the Faraday constant, z=2 in case of hydrogen but z=4 in case of oxygen. The conveying process at constant current consists of three phases. **Phase 1:** By the action of a constant gas production rate, the aggregate consisting of the two pistons 9 and 10 and the included medium 2 moves towards the mouth 1 of the cylinder. Thus an equal rate of medium 1 leaves the mouth and is injected into the client. **Phase 2:** The piston 9 has reached it's end-position. The pressure, P2, in compartment 2 increases proportional to the time until the opening pressure of valve 52 has been reached: $P_2 = P_1 + \Delta\pi_v$. This process determines a pause in which no medium is conveyed to the client; the end of the pause, $\tau$, is marked by the opening of the valve 52. Therefore is

$$\tau = (t_1/P_1) * \Delta\pi_v,$$

$t_1$ is the time that was needed to convey medium 1 at the constant pressure $P_1$ into the client. **Phase 3:** After valve 52 has opened, medium 2 is forced to flow with a reduced but constant flow rate into the client. If the pressure of the gas is now $P_4'$, the flow rate is reduced by a factor of $P_4/P_4'$ since $P_4' > P_4$.

It is possible to use any mechanic valve 52 to close and open the by-pass 51 according to the invention. Another embodiment of the invention comprises a membrane as valve 52 in by-pass 51 that bursts as soon as the differential pressure at piston 9 reaches the burst pressure, $\Delta\pi_b$. The bursting of the membrane changes phase 3 of the conveying process. After the pause and after opening of by-pass 51, the flow starts again with an increased rate compared to the flow rate in phase 1 but decreases afterwards steadily to the former value observed in phase 1.

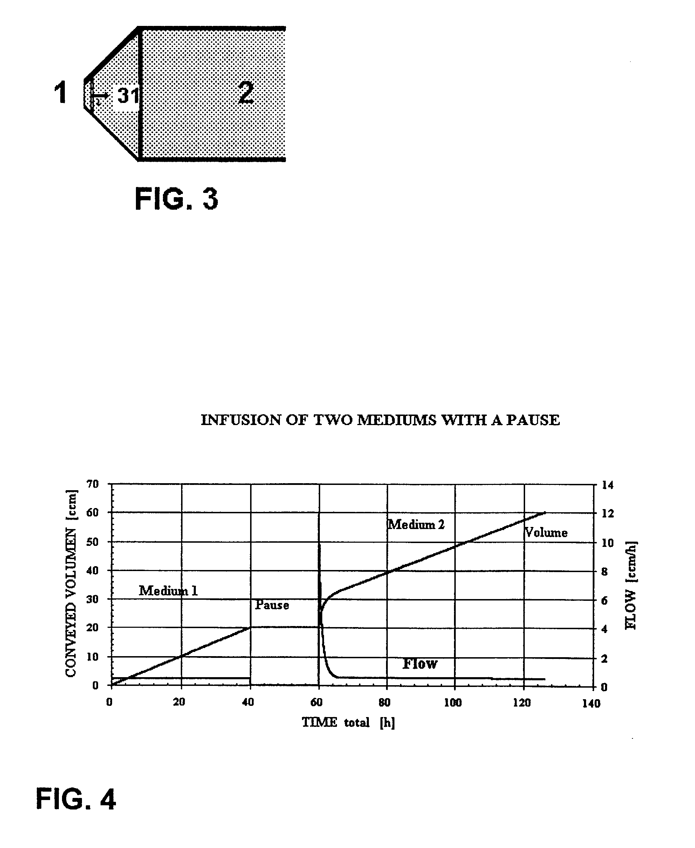

In another embodiment of the invention the bursting of the membrane can be supported by a knife or a head 31 that is located near to the mouth 1 as shown in FIG. 3. An example of the conveying process is displayed in FIG. 4. It starts with medium 1 that is infused during 40 hours. After a pause of 20 hours the infusion of the second medium starts. This third phase runs 66 hours. In this example the temperature is 30 °C and the atmospheric pressure is 1015 hPa. The hydrogen pressure during phase 1 is 1037 hPa but increases to 1550 hPa at the end of the pause. After bursting of the membrane the pressure decreases to 1037 hPa again within 4 hours.

In another embodiment of the invention, the valve 52 in by-pass 51 consists of an aperture made from an material that is not wetted by the conveyed liquids. We call this material 'hydrophobic' or lyophobic. A hydrophobic material is poorly wetted by water. A lyophobic aperture shows a capillary depression as action on the liquid. As an example, the aperture may consist of a porous disc made from PE (Polyethylen) or PTFE (Polytetrafluorethylen) with one single hole in it. An overpressure $\Delta\pi_b = (\sigma/r) * \cos\Theta$ is necessary to convey the liquid through this aperture. $\sigma$ is the surface tension ($\sigma = 72$ dyn/cm), r the capillary radius and $\Theta$ the wetting angel. The same equation is valid if the aperture consist of a number of parallel holes in a hydrophobic membrane or if the membrane consists of a porous lyophobic material with an suitable mean pore radius, r. The lyophobe aperture may also consist of a thin PTFE tube embedded within a tight material like epoxy-resin. For example: the capillary depression of water in a hole of r=0.01 cm radius and $\cos\Theta = -1$ is $\Delta\pi_b = 14,4$ mbar = 14,4 hPa. Decreasing the pore radius to 10 µm in a porous material increases $\Delta\pi_b$ to 144 hPa.

To avoid intermixing of the two conveyed media, the aperture can be closed by a plug made from Vaseline or the like that is pushed out by the pressurised medium 2 at the pressure difference $>= \Delta\pi_b$.

**Claims**

1. An apparatus for conveying successively two fluid mediums, especially two drugs, to a human or to an animal client comprising:

a cylinder 12 with a mouth 1 and an end-plate 13, therein a first piston 9 near to the mouth 1 and a second piston 10 near to the end-plate 13, thus defining three partitions within the cylinder:

⇒ partition one that is situated between mouth 1 and piston 9 and contains the first medium,
⇒ partition two that is situated between piston 9 and piston 10 and contains the second medium,
⇒ partition three that is situated between piston 10 and endplate 13 and takes up the driving gas that is generated within end-plate 13 by an electrolytic process, additionally comprising piston 9 to contain a by-pass 51 and a valve 52 within by-pass 51.

**2.** An apparatus for conveying successively two fluid mediums to a client, said apparatus consisting of a cylinder 12 with a mouth 1 and an end-plate 13 and a first piston 9 and a second piston 10 within said cylinder according to claim 1 comprising:
an end-plate 13 that contains one or more cells for the continuous development of hydrogen and/or oxygen gas in a rate that is determined by the electric current flowing through the cells.

**3.** An apparatus for conveying of two fluid mediums to a client according to claim 1 and 2 that consists of a cylinder 12 with a mouth 1 and an end-plate 13 and a first piston 9 with a by-pass 51 and a valve 52:
said valve comprising a sufficient small hole within a hydrophobic membrane.

**4.** An apparatus for conveying successively two fluid mediums to a client according to claim 1, 2 and 3 that consists of a cylinder 12 with a mouth 1 and an end-plate 13 and a first piston 9 with a by-pass 51 and a valve 52:
said valve comprising a burst membrane.

**5.** An apparatus for conveying of two fluid mediums, especially of two drugs, to a human or to an animal client consisting of a cylinder 12 with a mouth 1 and an end-plate 13, therein a first piston 9 near to the mouth 1 and a second piston 10 near to the end-plate 13, said piston 9 with a by-pass 51 and a valve 52 within said by-pass, comprising a knife or a head 31 integrated in mouth 1 for the perforation of the burst membrane.

**6.** An apparatus for conveying successively two fluid mediums to a client according to claim 1, 2 and 3 that consists of a cylinder 12 with a mouth 1 and an end-plate 13 and a first piston 9 with a by-pass 51 and a valve 52 comprising one single hole or a plurality of sufficient small holes within a hydrophobic membrane, the holes being plugged by a viscous

material like vaseline.

FIGURES:

**FIG 1**

**FIG. 2**

**FIG. 3**

INFUSION OF TWO MEDIUMS WITH A PAUSE

**FIG. 4**

European Patent
Office

## EUROPEAN SEARCH REPORT

Application Number

EP 98 12 4364

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | DE 41 21 351 A (WINSEL AUGUST) 14 January 1993 * the whole document * --- | 1,2 | A61M5/19 A61M5/315 |
| X | FR 2 110 516 A (BLANCHET HENRY) 2 June 1972 | 5 | |
| Y | * page 4, line 11 - page 5, line 24; figures 1-4 * | 1,2 | |
| A | --- | 4 | |
| Y | US 5 298 024 A (RICHMOND FRANK) 29 March 1994 * column 5, line 3 - column 8, line 52; figures 2-4 * --- | 1,2 | |
| A | US 3 911 916 A (STEVENS PETER A) 14 October 1975 * abstract; figure 4 * --- | 1,5 | |
| A | US 5 102 388 A (RICHMOND JOHN E) 7 April 1992 * abstract; figure 1 * --- | 1,5 | **TECHNICAL FIELDS SEARCHED** (Int.Cl.6) |
| A | US 4 439 184 A (WHEELER ROBERT P) 27 March 1984 * abstract; figures 1-5 * ----- | 1 | A61M |

The present search report has been drawn up for all claims

| Place of search | Date of completion of the search | Examiner |
|---|---|---|
| BERLIN | 27 April 1999 | Jameson, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 98 12 4364

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-1999

| Patent document cited in search report | | Publication date | Patent family member(s) | Publication date |
|---|---|---|---|---|
| DE 4121351 | A | 14-01-1993 | NONE | |
| FR 2110516 | A | 02-06-1972 | NONE | |
| US 5298024 | A | 29-03-1994 | NONE | |
| US 3911916 | A | 14-10-1975 | NONE | |
| US 5102388 | A | 07-04-1992 | NONE | |
| US 4439184 | A | 27-03-1984 | NONE | |

EPO FORM P0459

For more details about this annex : see Official Journal of the European Patent Office, No. 12/82